# EUROPEAN PATENT SPECIFICATION

(11) **EP 2 207 790 B1**
(45) Date of publication and mention of the grant of the patent: **21.08.2013**
(21) Application number: 08829747.8
(22) Date of filing: 05.09.2008
(51) Int. Cl.: C07K 5/06, C07K 5/08, C07K 1/16, C07K 1/20, C40B 40/10, C12N 5/00

(54) **BIOLOGICALLY ACTIVE C-TERMINAL ARGININE-CONTAINING PEPTIDES**
BIOLOGISCH AKTIVE PEPTIDE, DIE AM C-TERMINUS ARGININ ENTHALTEN
PEPTIDES C-TERMINAUX BIOLOGIQUEMENT ACTIFS CONTENANT DE L'ARGININE

(30) Priority: 05.09.2007 US 967644 P
(43) Date of publication of application: 21.07.2010
(73) Proprietor: Genentech, Inc., South San Francisco, CA 94080 (US)
(72) Inventor: WILKINS, James, San Mateo, CA 94402 (US); SHIRATORI, Masaru, Ken, Burlingame, CA 94010 (US); BREECE, Tim, San Francisco, CA 94123 (US)
(74) Representative: Keirstead, Tanis Evelyne
(86) International application number: PCT/US2008/075403
(87) International publication number: WO 2009/033024

(56) References cited:
- EP-A- 0 810 283
- US-A1- 2004 138 428
- "BD Bionutrients Technical Manual" October 2006 (2006-10), BD BIOSCIENCE , USA , XP002507461 page 36 - page 39
- JAN D C ET AL: "Peptone, a low-cost growth-promoting nutrient for intensive animal cell culture." CYTOTECHNOLOGY 1994, vol. 16, no. 1, 1994, pages 17-26, XP008099576 ISSN: 0920-9069
- SLOOTSTRA J W ET AL: "STRUCTURAL ASPECTS OF ANTIBODY-ANTIGEN INTERACTION REVEALED THROUGH SMALL RANDOM PEPTIDE LIBRARIES" MOLECULAR DIVERSITY, ESCOM SCIENCE PUBLISHERS, LEIDEN, NL, vol. 1, no. 2, 1 January 1995 (1995-01-01), pages 87-96, XP008065429 ISSN: 1381-1991

## Description

### FIELD OF THE INVENTION

The present invention concerns the separation, identification and characterization of active peptide fragments from peptones.

### BACKGROUND OF THE INVENTION

Hydrolysates of animal and plant tissues are often added to culture media for prokaryotic and eukaryotic cells to boost viable cell number and recombinant protein product titer. Thus, peptones from diverse sources have been widely employed in cell culture medium formulations to enhance cell culture growth, longevity and productivity. However, their presence as media additives for production of therapeutic proteins presents several challenges due to their undefined nature.

The use of animal-derived components as media additives for production of therapeutic proteins by mammalian cells can cause viral, mycoplasma and prion contaminations (Kallel et al., J. Biotechnol. 95:195-204 (2002)). The effort of avoiding the use of animal-derived components in cell culture media is supported by both European and American regulator authorities (Castle and Roberston, Dev. Biol. Stand. 99:191-196 (1999)). Animal serum has been successfully eliminated from media in the past decades and most of the media currently used is certified as protein-free (Froud, Dev. Biol. Stand. 99:157-166 (1999)). However, the switch to protein-free media often results in a loss of cell growth and productivity (Lee et al., J. Biotechnol. 69:85-93 (1999)).

As a result, protein hydrolysates (also known as peptones) arc frequency used in cell culture media as nutrient additives because some of them can be considered protein-free 25 (Burteau et al., In Vitro Cell. Dev. Biol. - Anim. 39(7):291-296 (2003), US-A1-2004/0138428, EP-A2-0810283 and "BD Bionutrients Technical Manual" Third Edition Revised (2006) BD BIOSCENCE). Peptones for cell culture are typically manufactured by enzymatic digestion of a variety of biologically based starting materials: animal tissues, milk-derived products, microorganisms or plants.

The general effect of peptones is an enhancement of cell growth and an increase of productivity, with the same product quality as compared to standard medium (Jan et al., Cytotechnology 16:17-26 (1994); Heidemann et al., Cytotechnology 32:157-167 (2000); Sung et al., Appl. Microbiol. Biotechnol. 63:527-536 (2004)). Several papers have shown that peptones contain materials such as free amino acids, oligopeptides. iron salts. lipids and trace elements (Franck et al., Biotechnol. Prog. 16:688-692 (2000); Martone et al., Bioresour. Technol. 96:383-387 (2005)). One study investigated the potential roles of peptones and showed that they can have a nutritional effect (Heidemann et al., Cytotechnology 32:157-167 (2000). Also, Schlaeger, J. Immunol. Meth. 194:191-199 (1996), demonstrated that a particular protein hydrolysate displays anti-apoptosis properties.

Despite the numerous investigations on peptone effects in cell culture, their mechanism of action is still unknown. And even though peptones have been shown to be beneficial in cell culture processes their usage also has many disadvantages. First, since some peptones are animal-derived, the similar problems are encountered as with secrum usage. In addition, peptones from other sources may also be a potential source of adventitious agents. Second, the uncertainty of the "active" components in peptone and their mechanism of action makes it difficult if not impossible to have a measure of quality on peptone lots used in cell culture manufacturing processes. Finally, because source materials and processes in the manufacture of peptone are not standardized or universally interchangeable, the peptones themselves are by nature a single-sourced raw material. Therefore, there is the opportunity to better characterize peptones in order to reduce or eliminate these disadvantages.

It would, therefore, be advantageous to understand the composition and identity of the active components in peptones in order to create "defined media," comprising well-specified active components in well-defined, reproducible concentrations. In addition the identification of active peptone components is expected to lead to a better understanding of the physiology of recombinant host cells and cell lines by opening a window to the control of cell growth and death and better control of recombinant protein expression.

Therefore, efforts have developed to characterize peptone compositions and to understand the mechanisms of their effect on cell cultures.

The present invention is based on the fractionation of an animal-derived peptone, PP3 into identifiable components and, by applying modern analytical techniques including high resolution mass spectrometry, to study the nature of these compounds. The present invention is based, at least in part, on the recognition that di- and tripeptides terminating in arginine play a role in the growth- and titer-promoting activities of PP3.

### SUMMARY OF THE INVENTION

The present invention is as set out in the claims.

### BRIEF DESCRIPTION OF THE DRAWINGS

Figure 1 is a schematic illustration of PP3 fractionation and analysis, as described in the Example.
Figure 2 shows the results of C18 reverse phase chromatography.
Figure 3 shows the results of chromatography of C18 flowthrough (unbound) fraction. "Relative specific activity" is defined as the activity of an equal weight of the fraction divided by that of PP3.
Figures 4A and 4B show that the specific activity of the G15- purified material (fractions F3-F7) was significantly increased over that of PP3 with respect to its ability to increase both recombinant protein titer (Figure 3A) and viable cell count (VCC; Figure 3B).
Figure 5. Mass spectral analysis of G-15 fraction. Top panel: MS scan of the fraction. Bottom Panels: ms/ms analysis of m/z 403.2, 389.2 and 375.2 respectively.
Figure 6. Frequency distribution of peptides in an *in silico* tripeptide library terminating in R. X axis: mass of tripeptides; Y axis: frequency at each mass.

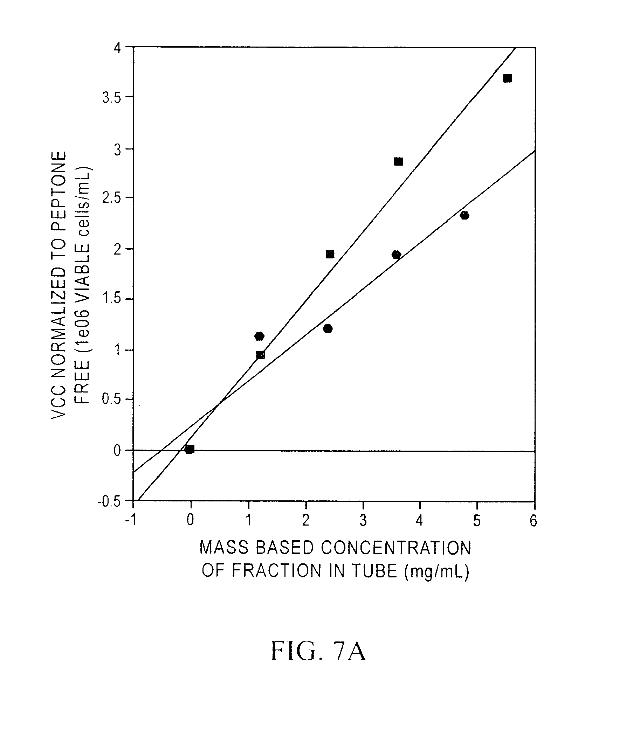

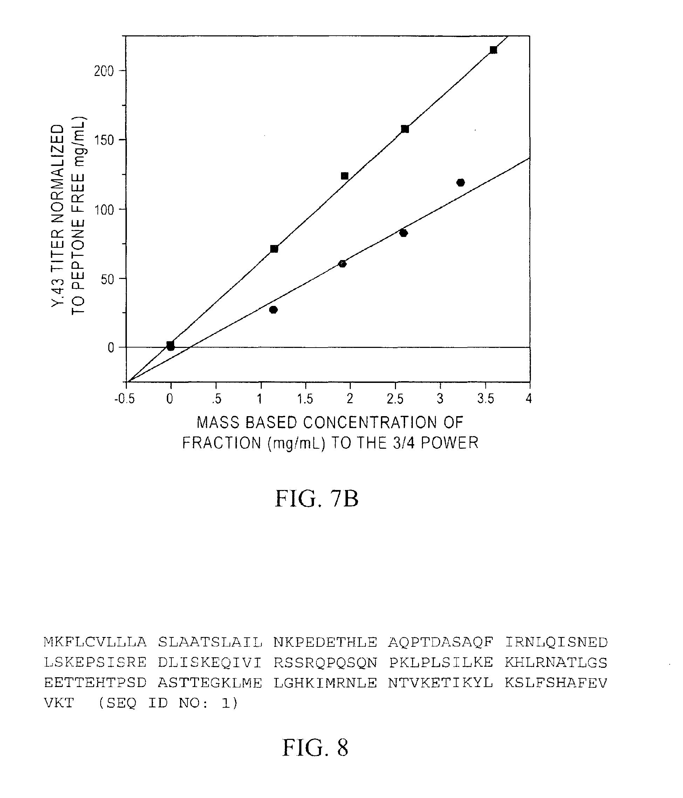

Figures 7A and B. Effects of PP3 and XXR peptides on VCC and product titer. A. VCC measured in the bioassay in response to addition of increasing concentrations of PP3 (Black line) or XXR peptides (Red line). B. Product Titer measured in the bioassay in response to addition of increasing concentrations of P33 (Black line) or XXR peptides (Red line).
Figure 8. Amino acid sequence of bovine Component-3 of proteose peptone (PP3) polypeptide (SEQ ID NO: 1).

### DETAILED DESCRIPTION OF THE INVENTION

### A. Definitions

Unless defined otherwise, technical and scientific terms used herein have the same meaning as commonly understood by one of ordinary skill in the art to which this invention belongs. Singleton *et al*., Dictionary of Microbiology and Molecular Biology 2nd ed., J. Wiley & Sons (New York, NY 1994) provides one skilled in the art with a general guide to many of the terms used in the present application.

The term "peptone" is used herein in the broadest sense and includes water-soluble protein derivatives obtained by partial hydrolysis of proteins, and mixtures thereof, including, without limitation, hydrolysate mixtures derived from the mucosal tissue of swine, cattle, and other animals, produced by hydrolysis with proteolytic enzymes, and well as hydrolysates from various plant tissues. The term specifically includes, without limitation, Component-3 of proteose peptone (PP3), also called lactophorin, which is a minor phosphoglycoprotein of 153 residues (Sorensen and Sorensen, J. Dairy Res. 60:535-542 (1993)) found in bovine milk (accession number in SwissProt databank: P80195; GenBank Accession No. CAA58309), and homologous proteins characterized in milk of other species as camel (Beg et al., FEBS Lett. 216:270-274 (1987), Ilama (Cantisani et al., J. Biochem. Biophys. Methods 21:227-236 (1990), ewe, and goat (Sorensen et al., J. Dairy Sci. 80:3176-3181(1997) and Lister et al., J. Dairy Res. 81:2111-2115 (1998). Up to now, PP3 has not been found in human milk. PP3 is available from commercial sources, such as, for example, Difco Laboratories, Detroit, Michigan.

The term "peptide" is used herein to refer to a compound containing two or more amino acids in which the carboxyl group of one acid is linked to the amino group of the other by an amide or "peptide" bond. The definition specifically includes peptides formed of at least two amino acid residues, and in particular di- and tri-peptides.

By "protein" is meant a sequence of amino acids for which the chain length is sufficient to produce the higher levels of tertiary and/or quaternary structure. Typically, the protein herein will have a molecular weight of at least about 15-20 kD, preferably at least about 20 kD. Examples of proteins encompassed within the definition herein include all mammalian proteins, in particular, therapeutic and diagnostic proteins, such as therapeutic and diagnostic antibodies, and, in general proteins that contain one or more disulfide bonds, including multi-chain polypeptides comprising one or more inter- and/or intrachain disulfide bonds.

The term "amino acid" or "amino acid residue" typically refers to an amino acid having its art recognized definition such as an amino acid selected from the group consisting of: alanine (Ala); arginine (Arg); asparagine (Asn); aspartic acid (Asp): cysteine (Cys): glutamine (Gln); glutamic acid (Glu); glycine (Gly); histidine (His); isoleucine (Ile): leucine (Leu); lysine (Lys); methionine (Met); phenylalanine (Phe); proline (pyro); scrinc (Ser); tlrreonine (Thr); tryptophan (Trp); tyrosine (Tyr); and valine (Val) although modified, synthetic, or rare amino acids array be used as desired. Thus, modified and unusual amino acids listed in 37 CFR 1.822(b)(4) are included within this definition and expressly incorporated herein by reference. Amino acids can be subdivided into various sub-groups. Thus, amino acids can be grouped as having a nonpolar side chain (e.g., Ala, Cys, Ile, Leu, Met, Phe, Pro, Val); a negatively charged side chain (e.g., Asp, Glu); a positively charged side chain (e.g., Arg, His, Lys); or an unchanged polar side chain (e.g., Asn, Cys, Gln, Gly, His, Met, Phe, Ser, Thr, Trp, and Tyr). Amino acids can also be grouped as small amino acids (Gly, Ala), nucleophilic amino acids (Ser, His, Thr, Cys), hydrophobic amino acids (Val, Leu, Ile, Met, Pro), aromatic amino acids (Phe, Tyr, Trp, Asp, Glu), amides (Asp, Glu), and basic amino acids (Lys, Arg).

The term "biological activity" or "peptone biological activity" in relation to the peptides described wherein is used to refer to any biological activity known to he exhibited by peptones, including, without limitation, the ability to promote cell growth and/or cell density and/or cell viability, and/or production efficiency of heterologous polypeptide in recombinant host cultures.

The term "chromatography" refers to the process by which a solute of interest in a mixture is separated from other solutes in a mixture as a result of differences in rates at which the individual solutes of the mixture migrate through a stationary medium under the influence of a moving phase, or in bind and elute processes.

The term "affinity chromatography" and "protein affinity chromatography" are used interchangeably herein and refer to a protein separation technique in which a protein of interest or antibody of interest is reversibly and specifically bound to a biospecific ligand. Preferably, the biospecific ligand is covalently attached to a chromatographic solid phase material and is accessible to the protein of interest in solution as the solution contacts the chromatographic solid phase material. The protein of interest (e.g., antibody, enzyme, or receptor protein) retains its specific binding affinity for the biospecific ligand (antigen, substrate, cofactor, or hormone, for example) during the chromatographic steps, while other solutes and/or proteins in the mixture do not bind appreciably or specifically to the ligand. Binding of the protein of interest to the immobilized ligand allows contaminating proteins or protein impurities to be passed through the chromatographic medium while the protein of interest remains specifically bound to the immobilized ligand on the solid phase material. The specifically bound protein of interest is then removed in active form from the immobilized ligand with low pH, high pH, high salt, competing ligand, and the like, and passed through the chromatographic column with the elution buffer, free of the contaminating proteins or protein impurities that were earlier allowed to pass through the column. Any component can be used as a ligand for purifying its respective specific binding protein, e.g. antibody.

The terms "non-affinity chromatography" and "non-affinity purification" refer to a purification process in which affinity chromatography is not utilized. Non-affinity chromatography includes chromatographic techniques that rely on non-specitic interactions between a molecule of interest (such as a protein, *e.g*. antibody) and a solid phase matrix.

A "cation exchange resin" refers to a solid phase which is negatively charged, and which thus has free cations for exchange with cations in an aqueous solution passed over or through the solid phase. A negatively charged ligand attached to the solid phase to form the cation exchange resin may, *e.g*., be a carboxylate or sulfonate. Commercially available cation exchange resins include carboxy-methyl-cellulose, sulphopropyl (SP) immobilized on agarose (*e.g*. SP-SEPHAROSE FAST FLOW™ or SP-SEPHAROSE HIGH PERFORMANCE™, from Pharmacia) and sulphonyl immobilized on agarose (*e.g*. S-SEPHAROSE FAST FLOW™ from Pharmacia). A "mixed mode ion exchange resin" refers to a solid phase which is covalently modified with cationic, anionic, and hydrophobic moieties. A commercially available mixed mode ion exchange resin is BAKERBOND ABX™ (J.T. Baker, Phillipsburg, NJ) containing weak cation exchange groups, a low concentration of anion exchange groups, and hydrophobic ligands attached to a silica gel solid phase support matrix.

The term "anion exchange resin" is used herein to refer to a solid phase which is positively charged, *e.g*. having one or more positively charged ligands, such as quaternary amino groups, attached thereto. Commercially available anion exchange resins include DEAE cellulose, QAE SEPHADEX™ and FAST Q SEPHAROSE™ (Pharmacia).

### B. Detailed Description

### Fractionation and analysis of peptones

The present invention is based on the identification of active peptone fragments, using a variety of chromatography and other separation techniques.

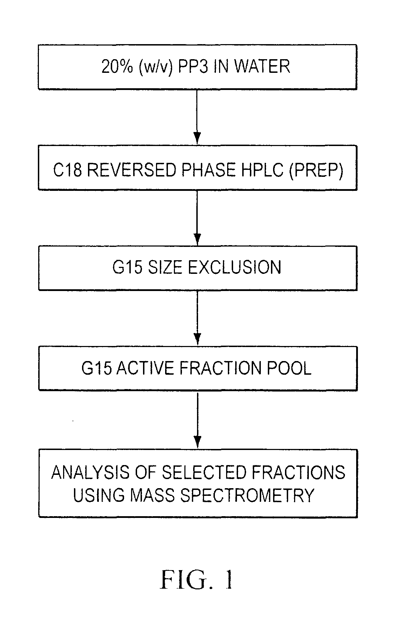

Fractionation of peptones can be performed by techniques well known in the art, including a combination of chromatographic steps. A typical scheme for the fractionation and analysis of the peptone PP3 is illustrated in Figure 1. The first step in this scheme is reversed phase HPLC (RP HPLC), which serves to separate components based on their hydrophobic character. Compounds bind to reversed phase HPLC columns in high aqueous mobile phase and are eluted from RP HPLC columns with high organic mobile phase. Thus, peptides can be separated using this technique by running linear or nonlinear gradients of an appropriate organic solvent.

The flow-through fraction from the reversed phase HPLC can then be subjected to further chromatographic separation steps, such as size-exclusion chromatography, as shown in Figure 1. Size exclusion chromatography (SEC) relies on sorting of molecules of different hydrodynamic radius based on the time these molecules spend within a chromatographic column. Partitioning occurs as a result of the molecules spending more or less time within the volume of the stationary phase which makes up the column. Molecules with large hydrodynamic radius elute earlyduring the separation; molecules with smaller radii elute later. SEC resins are well known in the art and are commercially available from a variety of manufacturers. In the experiments described in the Example below, SEPHADEX™ G15 size exclusion column (Amersham Biosciences) was used, but other size exclusion columns are also suitable.

If desired, peptones can be sub-fractionated by mixed-mode chromatography. In this technique, the stationary phase contains two distinct binding domains in a single chromatographic ligand. The separation principles commonly coupled are ion-exchange and RP chromatography. The application of mixed-mode chromatography is generally required when reverse phase chromatography (RP chromatography) fails to separate structurally close peptides. Currently, several manufacturers offer mixed-mode resins commercially that show different specificities, including the Hypercarb resin (Thermo Scientific), Oasis® HLB resin (Waters Corporation) and the Dowex Optipore® SD-2 resin (Dow Chemical Company). The Oasis sorbent is a copolymer of divinylbenzene and N-vinyl pyrrolidone. The hydrophilic-lipophilic-balanced composition allows for strong RP retention and improved wetting of the pores relative to traditional RP resins. Therefore, both polar and non-polar compounds can be adsorbed. The average size of the Oasis beads is 30 µm. The composition of the Optipore resin is similar to that of the Oasis resin (divinylbenzene with tertiary amines) giving it properties of both RP and a weak anion-exchange absorber. The particles average size is a few hundred µm.

At each purification step, the fractions obtained can be analyzed for biological activity and/or subjected to various analytical methods in order to determine their composition.

### Assays of peptide Activity

The peptone fractions generated can be tested using known bioassays, including a cell based bioassay described in the Example. Assays of the biological activity of peptone-derived peptides are typically based on the measurement of various culture parameters in cell cultures including the peptide in question in the culture medium. Such parameters include, for example, cell density, viable cell count, productivity of a heterologous polypeptide (e.g. antibody) produced, etc. Such assays are disclosed, for example, in Franek et al., Biotechnol. Prog. 16:688-692 (2000); Franek et al., Biotechnol. Prog. 18:155-158 (2002); Franek et al., Biotechnol. Prog. 19:169-174 (2003); Franek et al., Biotechnol. Prog. 21:96-08 (2005).

### Determination of the Composition of Peptone Fractions

Thus, the compositions of peptones and peptone fractions can be analyzed by mass spectrometry (MS), NMR, ICPMS, amino acid analysis, and various combinations of these and other techniques well known in the art.

Mass spectrometers consist of an ion source, mass analyzer, ion detector, and data acquisition unit. First, the peptides are ionized in the ion source. Then the ionized peptides are separated according to their mass-to-charge ratio in the mass analyzer and the separate ions are detected. Mass spectrometry has been widely used in protein analysis, especially since the invention of matrix-assisted laser-desorption ionisation/time-of-flight (MALDI-TOF) and electrospray ionisation (ESI) methods. There are several versions of mass analyzer, including, for example, MALDI-TOF and triple or quadrupole-TOF, or ion trap mass analyzer coupled to ESI. Thus, for example, a Q-Tof-2 mass spectrometer utilizes an orthogonal time-of-flight analyzer that allows the simultaneous detection of ions across the full mass spectrum range. For further details see, e.g. Chemusevich et al., J. Mass Spectrom. 36:849-865 (2001).

If desired, the amino acid sequences of the peptide fragments and eventually the proteins from which they derived can be determined by techniques known in the art, such as certain variations of mass spectrometry, or Edman degradation.

While the present invention is illustrated by peptide fragments identified in the degradation products of a particular peptone, it is expected that other peptones will have peptide components with similar structural and Functional characteristics.

### Combinatorial peptide libraries

Combinatorial peptide libraries are well known in the art, and methods for the generation and screening of such libraries arc described in standard textbooks, such as, for example, in Combinatorial Peptide Library Protocols (Methods in Molecular Biology), Shmuel Cabilly, ed., Humana Press Inc. 1998.
A peptide library is also described in Sloostra et al., Molecular Diversity, 1995, 1:87-96.

Combinatorial peptide libraries may be in different formats. For example, the so called "one bead one compound" libraries contain thousands of beads, each bearing multiple copies of a single library compound (Lam et al., Nature, 354, 82-84 (1991)). The library is screened for a desired activity and any active bead(s) is/are isolated. The active compound attached to the identified bead(s) identified is then characterized by conventional methods, such as, for example, by Edman degradation. Alternatively, a reductive approach may be employed. In another method, Houghten's positional fixing methodology (Bioorg. Med. Chem. Lett., 14, 1947-1951 (2004)), a set of related libraries is generated before screening. Each library contains compounds with one specific residue fixed as a single building block and the remaining residues fully randomized. Different libraries have a different building block at the fixed position. Screening this set of libraries, for a desired activity, enables direct identification of the optimum building block at the fixed residue. This process may be carried out sequentially, optimizing one residue at a time, or alternatively all of the sets of libraries may be screened simultaneously to allow the optimum library compound to be identified directly from a single round of screening.

Using the peptides specifically disclosed herein, the combinatorial peptide libraries can be used to identify additional peptides with improved chemical and/or biological characteristics.

### Peptide synthesis

The peptides identified as herein described can be synthesized by conventional methods of peptide synthesis, such as, for example, solid-phase synthesis methods, which can be performed in a variety of forms, such as, for example, using Fmoc (9H-fluoren-9-yl-methoxy-carbonyl) or Boc (tert-butoxycarbonyl) protecting groups to protect the N-termini of amino acid monomers used in the synthesis. Automated synthesizers arc commercially available for both techniques, but solid phase peptide synthesis can also be performed manually. For further details see, for example, Atherton, E., Sheppard, R.C. (1989). Solid Phase peptide synthesis: a practical approach. Ocford, England: IRL Press. ISBN 0199630674; and Steward, J.M., Young, J.D. (1984). Solid phase peptide synthesis, 2nd edition, Rockford: Pierce Chemical Company, 91. ISBN 0935940030.

### Uses of the di- and tri-peptides

The di- and tri-peptides described herein can be used as components of growth medina used for the production of recombinant polypeptides, including antibodies.

Recombinant polypeptides, such as antibodies, can be produced in a variety of cukaryotic and prokaryotic host organisms.

Suitable host cells for the expression of glycosylated polypeptide, such as antibodies, can be derived from multicellular organisms. Examples or invertebrate cells include plant and insect cells. Numerous baculoviral strains and variants and corresponding permissive insect host cells from hosts such as Spodoptera frugiperda (caterpillar), Acdcs aegypti (mosquito), Aedes albopictus (mosquito), Drosophila melanogaster (fruitfly), and Bombyx mori have been identified. A variety of viral strains for transfection arc publicly available, e.g., the L-1 variant of Autographa californica NPV and the Bm-5 strain of Bombyx mori NPV, and such viruses may be used as the virus herein particularly for transfection of Spodoptera frugiperda cells. Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed bySV40 (COS-7, ATCC CRL 1651); human embryonic kidney line (293 or 293 cells subloned for growth in suspension culture, Graham et al, J. Gen Virol. 36:59 (1977)); baby hamster kidney cells (BHK, ATCC CCL 10); Chinese hamster ovary cells/-DHFR (CHO, Urlaub ct al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL, 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442): human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Ilep G2).

For example, recombinant polypeptides, such as antibodies, can be produced in dp12.CHO cells, the production of which from CHO-K1 DUX-B11 cells as described in EP307247. CHO-K1 DUX-B11 cells were, in turn, obtained from CHO-K1 (ATCC No. CCL61 CHO-K1) cells, following the methods described in Simonsen. C. C., and Levinson, A. D., (1983) Proc. Natl. Acad. Sci. USA 80:2495-2499 and Urlaub G., and Chasin, L.. (1980) Proc. Natl. Acad. Sci USA 77:4216-4220. In addition, other CHO-K1 (dhfr⁻) cell lines arc known and can be used.

The mammalian host cells used to produce peptides, polypeptides and proteins can be cultured in a variety of media. Commercially available media such as Ham's F10 (Sigma), Minimal Essential Medium ((MEM), Sigma), RPM1-1640 (Sigma), and Dulbecco's Modified Eagle's Medium ((DMEM, Sigma) are suitable for culturing the host cells. In addition, any of the media described in Ham and Wallace (1979), Meth. in Enz. 58:44, Barnes and Sato (1980), Anal. Biochem. 102:255, U.S. Pat. No. 4,767,704; 4,657,866; 4,927,762; or 4,560,655; WO 90/03430; WO 87/00195; U.S. Pat. No. Re. 30,985; or U.S. Pat. No. 5,122,469, may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

The peptides described herein can be used, individually or in various combinations, as components of any commercially available or custom made culture medium.

The use of the peptides herein is not limited to the culturing of mammalian, or, in general cukaryotic host cells. The peptides described herein also find utility in cell cultures of prokaryotic host organisms. Exemplary prokaryotic host cells include, without limitation, eubacteria, such as Gram-negative or Gram-positive organisms, for example, Enterobacteriaccac such as Escherichia, e.g., E. coli, Enterobacter, Erwinia, Klebsiella, Proteus, Salmonella, e.g., Salmonella typhimurium, Serrafia, e.g, Serratia marcescans, and Shigeila, as well as Bacilli such as B. subtilis and B. licheniformis (e.g., B. licheniformis 41P disclosed in DD 266,710 published Apr. 12, 1989), Pseudomonas such as P. aeruginosa, and Streptomyces. One preferred E. coli cloning host is E. coli 294 (ATCC 31,446), although other strains such as E. coli B, E. coli X 1776 (ATCC 31,537), and E coil W3110 (ATCC 27,325) are suitable.

Further details of the present invention are provided in the following Example.

### Example

### Molecular identification of biologically active C-terminal arginine containing tripeptides using a combinatorial library

### Materials and Methods

### TubeSpin Bioassay

The TubeSpin bioassay was designed to examine the effects of a cell culture medium additive on cellular proliferation and productivity (as determined by viable cell density and concentration of antibody produced, respectively) in a 5 milliliter (mL) working volume using an initial viable cell density of 1.5 x 10^6 cells/mL.

Each culture was prepared in a volume of 7.15 mL from which 5 mL are transferred to the assay tube. The assay tube is a 50 mL centrifuge tube with a specialized cap that contains a membrane with a 0.2 micron pore-size to facilitate the flow of gases. The 2.15 mL that remained was used to measure viable cell density (to confirm that both the target density has been achieved and that the cells are viable), osmolality and metabolic data (pH, glutamine, glutamate, glucose, lactate, ammonium, sodium, potassium).

Depending upon the starting concentration and osmolality of the desired medium additive, a target concentration for that additive was determined. From this data, the volume of the additive required, the volume of sodium chloride required to achieve physiological osmolality in the final culture, and the volume of water necessary to achieve 7.15 mL are calculated. Using these values a preparative solution of medium additive, sodum chloride, and water was assembled.

Cells in Selective Genentech Essential Medium (GEM) were then medium-exchanged into a production-quality medium referred to as GEM2. Cells in GEM2 were then added to each preparative tube to achieve the stated 1.5 x 10^6 cells/mL in 7.15 mL. Each assay tube then received 5 mL and was incubated at 37 degrees centigrade for 4.75 days.

At the end of the incubation period the viable cell density, osmolality, and metabolic profile were once again measured, and a sample of the assay culture was sent to a separate antibody quantification assay to determine its concentration.

### Cl8 reversed phase preparative chromatography

A 20% (w/v) aqueous solution of PP3 was prepared and was used immediately. A 5 x 5 cm stainless steel column was packed with Waters Preparative C18 silica (125Å pore size; 55-105 micron particle size). The column was preequilibrated with 10 volumes of 0.02% (w/v) trifluoroacetic acid (TFA) in water (Buffer A) and was operated at a flowrate of 60 ml/min. 200mL of the above PP3 solution was loaded onto the column. Solvent control and effluent monitoring of the column was achieved using an Akta chromatography system controlled by Unicorn software (GE Healthcare). Following the load, the column was washed with buffer A until the absorbance of the effluent at 280nm reached baseline. The column was then washed with 85% (v/v) acetonitrile (ACN) containing 0.02% TFA to elute bound material. Fractions were assayed for biological activity using the bioassay

### G15 Sephadex preparative chromatography

The flowthrough material from the C18 was concentrated by rotary evaporation and was further fractionated on a G-15 Sephadex column (7cm dia. x 50 cm 1.). The column was equilibrated with deionized water prior to loading and the entire C18 flowthrough pool was loaded. Fractions were collected and assayed for activity using the bioassay described above.

### Hypercarb analytical chromatography

Samples of active fractions were analyzed using a Hypercarb (graphite) column (5 micron particle size; column dimensions: 0.1mm x 50mm). The buffers used were Buffer A: aqueous 0.02% TFA; and Buffer B: 85% ACN + 0.02% TFA. Various volumes (0-10 microliters) were loaded onto the column which was precquilibrated with Buffer A and eluted with a linear gradient going to 50% Buffer B over 25 minutes. The column was operated at a flowrate of 0.1ml/min. Solvent control was achieved with an Agilent 1100 HPLC equipped with diode array detector and an MSD single quadrapole mass spectrometer. The entire system was under the control of Agilent Chemstation software.

### Mass spectrometry

High resolution mass spectrometry was performed using a Q-TOF (Waters Corporation) or an Orbitrap mass spectrometer (Thermofinnigan). The instruments were operated in the static spray mode and mass accuracy was found to be <5 ppm in both cases. Spectra were used to obtain accurate mass information which was used to correlate with peptide library information.

### Bioinformatics

*In silico* libraries of dipeptides and tripeptides were constructed with the necessary mass accuracies to correlate with masses determined as discussed above.

### Peptide synthesis

A tripeptide library was created using Fmoc solid phase peptide synthesis. The library was designed to contain all possible tripeptides terminating in arginine (R), i.e. XXR.

### Results

### Fractionation of PP3

The scheme for PP3 fractionation and analysis is shown diagrammatically in Figure 1.

### C18 Reversed Phase HPLC

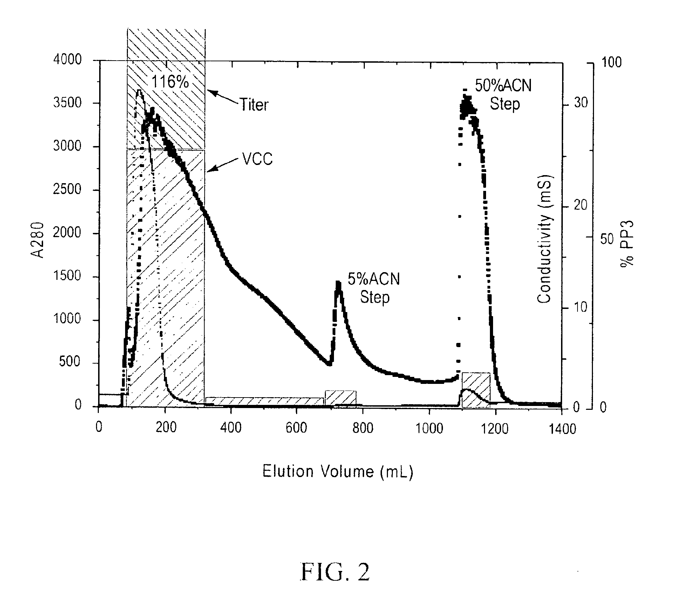

The initial fractionation of PP3 was accomplished using reversed phase HPLC. The chromatogram shown in Figure 2 is representative of the reversed phase chromatography profile of PP3. Most of the mass (~70% of the initial dry weight) flows through the column as does most of the biological activity.

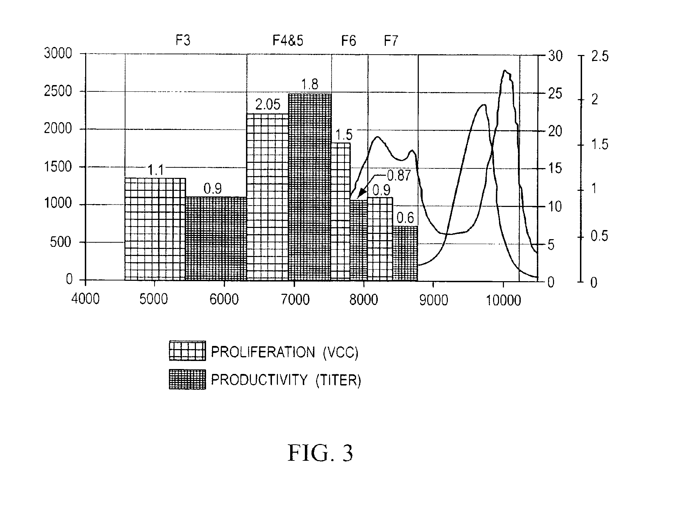

The flowthrough fraction from C18 was concentrated and loaded onto G-15 Sephadex for size exclusion chromatography, the results of which are shown in Figure 3.

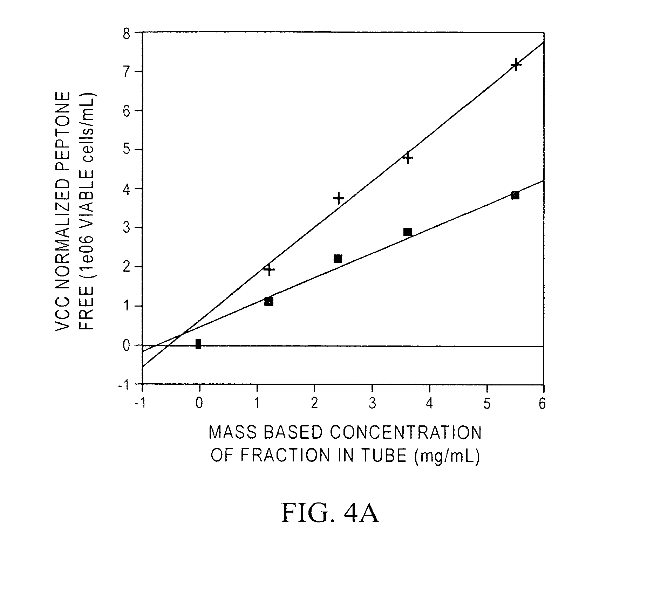

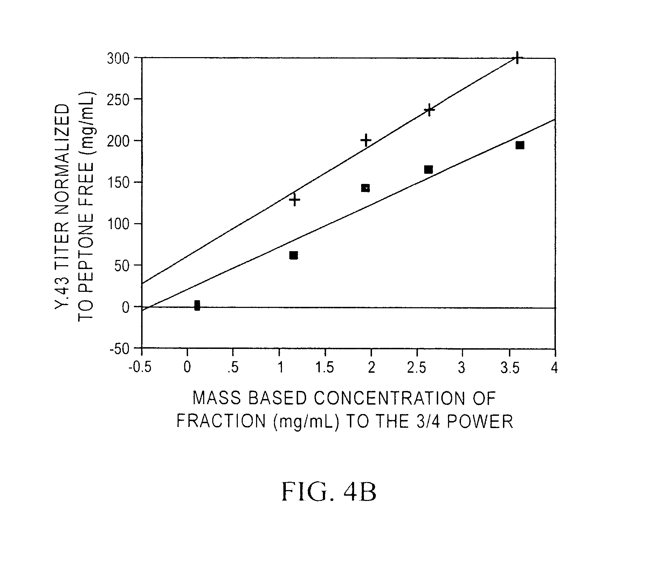

Fractions from the G15 column were analyzed for bioactivity. The specific activity of the G15- purified material (fractions F3-F7) was significantly increased over that of PP3 with respect to its ability to increase both recombinant protein titer (Figure 4A) and viable cell count (VCC; Figure 4B).

### Mass Spectrometry on G15 fraction pool

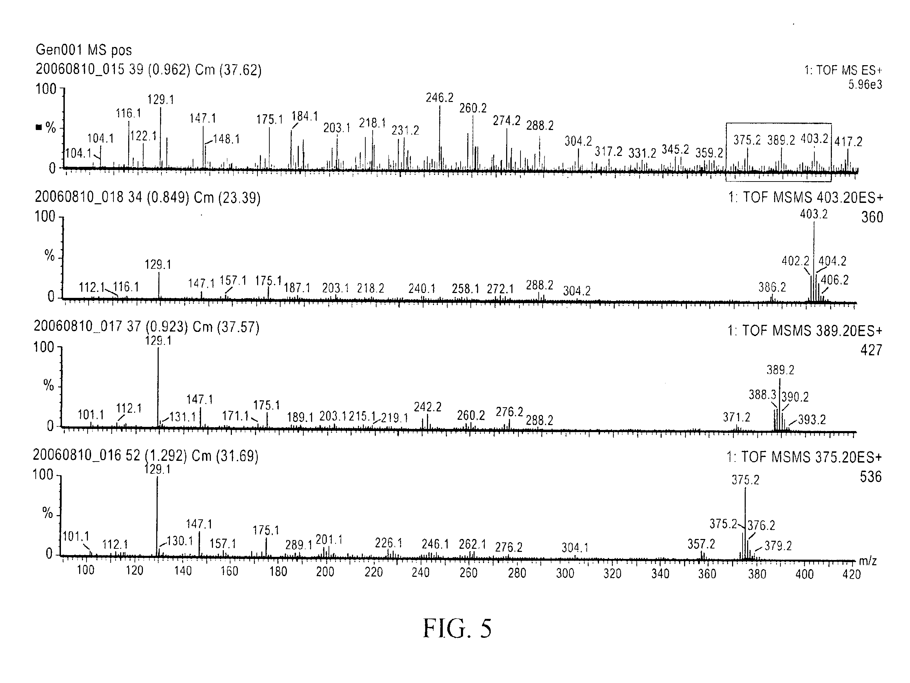

Active fractions were analyzed using mass spectrometry in order to obtain information about the molecular nature of the active components. Figure 5 shows a representative mass spectrum obtained from a sample of G15 pool obtained as described above. The spectrum contains numerous peaks with masses <500 Da. One striking feature of the spectra was the fact that they contained many peaks differing in mass by 14 mass units in a repeating fashion. Also, a peak at 175.1 m/z (Figure 5) was identified as arginine by exact mass and subsequent elemental composition analysis. Further, analysis of three of the masses (403.2, 389.2 and 375.2 m/z; marked by the box) from the spectrum shown in Figure 5 using collision induced dissociation (CID) revealed similar fragmentation patterns in the mass range <200 Da. This included the identification of an intact arginine residue at 175.1 m/z from each of the three masses. In addition, based on exact mass measurements, these molecules were identified as pcptidcs with the following possible identities:
403.2 Da: EVR; DIR; DLR
389.2 Da: LTR; ITR
375.2 Da: SIR; SLR; TVR

The order of the first 2 amino acids in the above sequences could also be reversed. Several possibilities exist at each mass because of the overlap in combinations of individual amino acids.

### Creation of an in silico library of tripeptides containing arginine

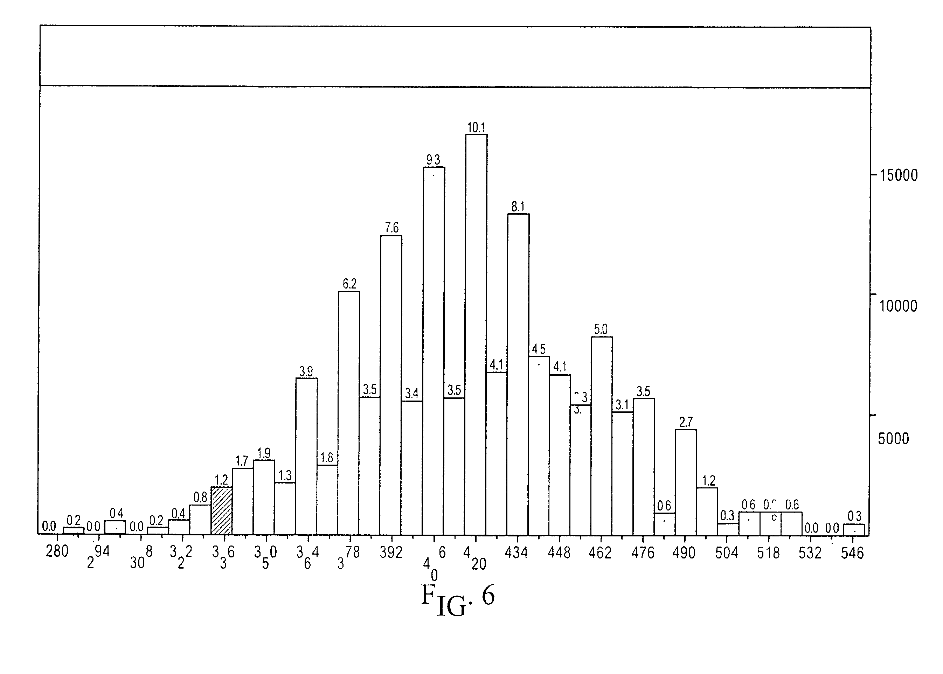

The 14 mass-unit repeating structure seen in mass spectra from peptone fractions *and* the presence of C-terminal arginine in a group of these molecules suggested the presence of an enzymatically-created "combinatorial library" of peptides in PP3 arising from the animal tissues. To further investigate this idea, we decided to create an *in silico* library of C-terminal arginine-containing tripeptides. The library contains 400 possible peptides and its mass composition and distribution is represented in the graph shown in Figure 6. the distribution indeed resembles the 14 mass-unit differences seen in the peptone fractions with repeating peaks at intervals of 14 Da.

### construction and analysis of a real XXR combinatorial peptide library

In order to further explore the idea that C-terminal arginine containing peptides might be responsible for some of the activity seen in PP3 fractions, we made a combinatorial library of these molecules using solid phase (Fmoc) peptide synthesis. The library containing lyophilized peptides was dissolved in deionized water and the pH of the solution was adjusted to ~ 7.0 with NaOH. The solution was then desalted using a G-15 column. A fraction of the library was collected, concentrated by rotary evaporation and assayed by mass spectrometry and by bioassay. Mass spectral analysis revealed the presence of both di- and tripeptides. Dipeptides found in the mixture were thought to arise from a certain proportion of failed peptide synthesis reactions.

Mass spectrometry revealed the presence of numerous peaks, which were compared with the *in silico* library referred to above. Table 1 lists the masses, identities and relative intensities of the peptides found in this fraction. The assignments are based on accurate mass measurements with errors <5ppm. This list represents a partial list of all the peptides present in this fraction.

### Bioassay of G-15 XXR fraction

The XXR peptides prepared as noted above were assayed for activity in the bioassay and found to have significant positive effects on both cell growth and titer.

### Summary

The results presented in this example show that PP3 contains active molecules with molecular masses <500 Da. Some of these molecules were identified as C-terminal arginine containing tripeptides. A combinatorial library of these peptides showed biological activity in a 6-day bioassay. We concluded that C-terminal arginine containing tripeptides arc responsible, at least in part, for the growth- and titer-promoting activities of PP3.

**TABLE 1**

| Peptide identifications from XXR | | | | | |
|---|---|---|---|---|---|
| XXRpeptides identified from G15 bioactive fraction | | | | | |
| # | Assignment | Theor m/z | Error (abs) | Error (ppm) | Rel-intensity |
| 1 | DIR | 403.2305 | 0.000408 | 1.011915 | 52 |
| 2 | DLR | 403.2305 | 0.000408 | 1.011915 | 52 |
| 3 | EVR | 403.2325 | 0.000408 | 1.011915 | 52 |
| 4 | IR | 288.2036 | 0.000365 | 1.266195 | 45 |
| 5 | LR | 288.2036 | 0.000365 | 1.266195 | 45 |
| 6 | DPR | 387.1992 | 0.000308 | 0.795164 | 38 |
| 7 | PR | 272.1723 | 0.000365 | 1.340232 | 38 |
| 8 | DR | 290.1464 | 0.000344 | 1.185539 | 19 |
| 9 | TVR | 375.2356 | 0.000293 | 0.781895 | 27 |
| 10 | EIR | 417.2462 | 0.000458 | 1.097935 | 27 |
| 11 | ELR | 417.2462 | 0.000458 | 1.097935 | 27 |
| 12 | VR | 274.1879 | 0.000415 | 1.513006 | 25 |
| 13 | SR | 262.1515 | 0.000429 | 1.637755 | 25 |
| 14 | AR | 246.1566 | 0.000415 | 1.6847 | 24 |
| 15 | ER | 304.1621 | 0.000294 | 0.966767 | 23 |
| 16 | INR | 402.2465 | 0.000392 | 0.975621 | 23 |
| 17 | LNR | 402.2465 | 0.000392 | 0.975621 | 23 |
| 18 | QVR | 402.2465 | 0.000392 | 0.975621 | 23 |
| 19 | AVR | 345.225 | 0.000229 | 0.662411 | 22 |
| 20 | GIR | 345.225 | 0.000229 | 0.662411 | 22 |
| 21 | GLR | 345.225 | 0.000229 | 0.662411 | 22 |
| 22 | MR | 306.16 | 0.000387 | 1.262895 | ? |
| 23 | IVR | 387.272 | 0.000379 | 0.97839 | 20 |
| 24 | LVR | 387.272 | 0.000379 | 0.97839 | 20 |
| 25 | ITR | 389.2512 | 0.000343 | 0.882384 | 19 |
| 26 | LTR | 389.2512 | 0.000343 | 0.882384 | 19 |
| 26 | DTR | 391.1941 | 0.000323 | 0.824468 | 17 |
| 28 | ESR | 391.1941 | 0.000323 | 0.824468 | 17 |
| 29 | GGR | 289.1624 | 0.000328 | 1.1356642 | 17 |
| 30 | NR | 289.1624 | 0.000328 | 1.135642 | 1 |
| 31 | DVR | 389.2149 | 0.000358 | 0.9197 | 17 |
| 32 | IIR | 401.2876 | 0.000429 | 1.069001 | 17 |
| 33 | ILR | 401.2876 | 0.000429 | 1.069001 | 17 |
| 34 | LLR | 401.2876 | 0.000429 | 1.069001 | 17 |
| 35 | AIR | 359.2407 | 0.000279 | 0.775956 | 16 |
| 36 | ALR | 359.2407 | 0.000279 | 0.775956 | 16 |
| 37 | ADR | 361.1836 | 0.000258 | 0.7138 | 16 |
| 38 | EGR | 361.1836 | 0.000258 | 0.7138 | 16 |
| 39 | AGR | 303.1781 | 0.000278 | 0.918467 | 16 |
| 40 | QR | 303.1781 | 0.000278 | 0.918467 | 15 |
| 41 | GR | 232.141 | 0.000465 | 2.001482 | 15 |
| 42 | DQR | 418.205 | 0.000422 | 1.008051 | 14 |
| 43 | ENR | 418.205 | 0.000422 | 1.008051 | 14 |
| 44 | DMR | 421.1869 | 0.00043 | 1.020357 | 13 |
| 45 | EMR | 435.2026 | 0.00048 | 1.102556 | 11 |
| 46 | ASR | 333.1886 | 0.000243 | 0.729836 | 11 |
| 47 | GTR | 333.1886 | 0.000243 | 0.729836 | 11 |
| 48 | FPR | 419.2407 | -0.003021 | -7.206469 | 11 |
| 49 | IMR | 419.2441 | 0.000351 | 0.836513 | 11 |
| 50 | LMR | 419.2441 | 0.000351 | 0.836513 | 11 |
| 51 | IPR | 385.2563 | 0.000329 | 0.853533 | 10 |
| 52 | LPR | 385.2563 | 0.000329 | 0.853533 | 10 |

## Claims

1. Use of a peptide of the formula
(X1)ₙX2R
wherein X1 and X2 may be identical or different and independently represent any amino acid other than arginine;
n is 0 or 1; and
R stands for arginine,
wherein said peptide exhibits a peptone biological activity, for promoting cell growth and/or cell density and/or cell viability, and/or production efficiency of heterologous polypeptides in recombinant host cultures.

2. The use according to claim 1, wherein said peptide is obtained by fractionation of a peptone or is chemically synthesized.

3. The use according to claim 2 wherein said peptone that is fractionated is a PP3 peptone (component-3 of protease peptidone).

4. The use according to claim 1 wherein said peptide is selected from the peptides DIR, DLR, EVR, DPR, TVR, EIR, ELR, INR, LNR, QVR, AVR, GIR, GLR, IVR, LVR, ITR, LTR, DTR, ESR, GGR, DVR, IIR, ILR, LLR, AIR, ALR, ADR, EGR, AGR, DQR, ENR, DMR, EMR, ASR, GTR, IMR, FPR, LMR, IPR, LPR, IR, LR, PR, DR, VR, SR, AR, ER, MR, NR, QR, and GR.

5. The use according to claim 1 wherein said peptide is a tripeptide.

6. The use according to claim 5 wherein said tripeptide is selected from the tripeptides-DIR, DLR, EVR, DPR, TVR, EIR, ELR, INR, LNR, QVR, AVR, GIR, GLR, IVR, LVR, ITR, LTR, DTR, ESR, GGR, DVR, IIR, ILR, LLR, AIR, ALR, ADR, EGR, AGR, DQR, ENR, DMR, EMR, ASR, GTR, IMR, FPR, LMR, IPR, and LPR.

7. The use according to claim 1 wherein said peptide is a dipeptide.

8. The use according to claim 7 wherein said dipeptide is selected from the dipeptides IR, LR, PR, DR, VR, SR, AR, ER, MR, NR, QR, and GR.

9. A combinatorial peptide library consisting of peptides of the formula
(X1)ₙX2R
wherein X1 and X2 may be identical or different and independently represent any amino acid other than arginine;
n is 0 or 1; and
R stands for arginine,
wherein at least some of said peptides exhibit a peptone biological activity, for promoting cell growth and/or cell density and/or cell viability, and/or production efficiency of heterologous polypeptides in recombinant host cultures.

## Patentansprüche

1. Verwendung eines Peptids der Formel
(X1)ₙX2R,
worin X1 und X2 gleich oder unterschiedlich sein können und unabhängig voneinander für eine beliebige andere Aminosäure als Arginin stehen;
n = 0 oder 1 ist; und
R für Arginin steht,
wobei das Peptid biologische Peptonaktivität aufweist, zur Förderung des Zellwachstums und/oder der Zelldichte und/oder der Zelllebensfähigkeit und/oder der Produktionseffizienz von heterologen Polypeptiden in rekombinanten Wirtskulturen.

2. Verwendung nach Anspruch 1, wobei das Peptid durch Fraktionierung eines Peptons erhalten wurde oder chemisch synthetisiert ist.

3. Verwendung nach Anspruch 2, wobei das fraktionierte Pepton ein PP3-Pepton (Komponente 3 von Proteasepeptidon) ist.

4. Verwendung nach Anspruch 1, wobei das Peptid aus den Peptiden DIR, DLR, EVR, DPR, TVR, EIR, ELR, INR, LNR, QVR, AVR, GIR, GLR, IVR, LVR, ITR, LTR, DTR, ESR, GGR, DVR, IIR, ILR, LLR, AIR, ALR, ADR, EGR, AGR, DQR, ENR, DMR, EMR, ASR, GTR, IMR, FPR, LMR, IPR, LPR, IR, LR, PR, DR, VR, SR, AR, ER, MR, NR, QR und GR ausgewählt ist.

5. Verwendung nach Anspruch 1, wobei das Peptid ein Tripeptid ist.

6. Verwendung nach Anspruch 5, wobei das Tripeptid aus den Tripeptiden DIR, DLR, EVR, DPR, TVR, EIR, ELR, INR, LNR, QVR, AVR, GIR, GLR, IVR, LVR, ITR, LTR, DTR, ESR, GGR, DVR, IIR, ILR, LLR, AIR, ALR, ADR, EGR, AGR, DQR, ENR, DMR, EMR, ASR, GTR, IMR, FPR, LMR, IPR und LPR ausgewählt ist.

7. Verwendung nach Anspruch 1, wobei das Peptid ein Dipeptid ist.

8. Verwendung nach Anspruch 7, wobei das Dipeptid aus den Dipeptiden IR, LR, PR, DR, VR, SR, AR, ER, MR, NR, QR und GR ausgewählt ist.

9. Kombinatorische Peptidbibliothek, die aus Peptiden der Formel
(X1)ₙX2R
besteht, worin X1 und X2 gleich oder unterschiedlich sein können und unabhängig voneinander für eine beliebige andere Aminosäure als Arginin stehen;
n = 0 oder 1 ist; und
R für Arginin steht,
wobei zumindest einige der Peptide biologische Peptonaktivität aufweisen, zur Förderung des Zellwachstums und/oder der Zelldichte und/oder der Zelllebensfähigkeit und/oder der Produktionseffizienz von heterologen Polypeptiden in rekombinanten Wirtskulturen.

## Revendications

1. Utilisation d'un peptide de formule
(X1)ₙX2R
dans laquelle X1 et X2 peuvent être identiques ou différents et représentent indépendamment n'importe quel aminoacide autre que l'arginine ;
n est égal à 0 ou 1 ; et
R représente l'arginine,
dans laquelle ledit peptide présente une activité biologique de peptone, pour activer la croissance cellulaire et/ou la densité cellulaire et/ou la viabilité cellulaire et/ou l'efficacité de production de polypeptides hétérologues dans des cultures d'hôtes recombinants.

2. Utilisation suivant la revendication 1, dans laquelle ledit peptide est obtenu par fractionnement d'une peptone ou est synthétisé chimiquement.

3. Utilisation suivant la revendication 2, dans laquelle ladite peptone qui est fractionnée est une peptone PP3 (constituant 3 de la protéase-peptidone).

4. Utilisation suivant la revendication 1, dans laquelle ledit peptide est choisi parmi les peptides DIR, DLR, EVR, DPR, TVR, EIR, ELR, INR, LNR, QVR, AVR, GIR, GLR, IVR, LVR, ITR, LTR, DTR, ESR, GGR, DVR, IIR, ILR, LLR, AIR, ALR, ADR, EGR, AGR, DQR, ENR, DMR, EMR, ASR, GTR, IMR, FPR, LMR, IPR, LPR, IR, LR, PR, DR, VR, SR, AR, ER, MR, NR, QR et GR.

5. Utilisation suivant la revendication 1, dans laquelle ledit peptide est un tripeptide.

6. Utilisation suivant la revendication 5, dans laquelle ledit tripeptide est choisi parmi les tripeptides DIR, DLR, EVR, DPR, TVR, EIR, ELR, INR, LNR, QVR, AVR, GIR, GLR, IVR, LVR, ITR, LTR, DTR, ESR, GGR, DVR, IIR, ILR, LLR, AIR, ALR, ADR, EGR, AGR, DQR, ENR, DMR, EMR, ASR, GTR, IMR, FPR, LMR, IPR et LPR.

7. Utilisation suivant la revendication 1, dans laquelle ledit peptide est un dipeptide.

8. Utilisation suivant la revendication 7, dans laquelle ledit dipeptide est choisi parmi les dipeptides IR, LR, PR, DR, VR, SR, AR, ER, MR, NR, QR et GR.

9. Banque de peptides combinatoire consistant en peptides de formule
(X1)ₙX2R
dans laquelle X1 et X2 peuvent être identiques ou différents et représentent indépendamment n'importe quel aminoacide autre que l'arginine ;
n est égal à 0 ou 1 ; et
R représente l'arginine,
dans laquelle au moins certains desdits peptides présentent une activité biologique de peptone, pour activer la croissance cellulaire et/ou la densité cellulaire et/ou la viabilité cellulaire et/ou l'efficacité de production de polypeptides hétérologues dans des cultures d'hôtes recombinants.
